(19) Europäisches Patentamt

European Patent Office

Office européen des brevets

(11) **EP 1 642 586 A1**

(12) **EUROPEAN PATENT APPLICATION**
published in accordance with Art. 158(3) EPC

(43) Date of publication:
 **05.04.2006 Bulletin 2006/14**

(51) Int Cl.:
 *A61K 35/12* (1985.01)   *A61K 38/39* (1995.01)
 *C12N 5/10* (1990.01)   *A61P 35/00* (2000.01)

(21) Application number: **04704767.5**

(22) Date of filing: **23.01.2004**

(86) International application number:
 **PCT/JP2004/000630**

(87) International publication number:
 **WO 2004/108144 (16.12.2004 Gazette 2004/51)**

(84) Designated Contracting States:
 **AT BE BG CH CY CZ DE DK EE ES FI FR GB GR HU IE IT LI LU MC NL PT RO SE SI SK TR**

(30) Priority: **06.06.2003 JP 2003162940**

(71) Applicants:
 • **Kringle Pharma Inc.**
  **Osaka 560-0082 (JP)**
 • **Nakamura, Toshikazu**
  **Kyoto 606-8333 (JP)**

(72) Inventors:
 • **NAKAMURA, Toshikazu**
  **Kyoto-shi, Kyoto 606-8333 (JP)**

 • **MATSUDA, Takehisa**
  **Higashi-ku Fukuoka-shi, Fukuoka 813-0044 (JP)**
 • **TANAKA, Masao**
  **Fukuoka-shi, Fukuoka 819-0374 (JP)**
 • **MANABE, Tatsuya**
  **Miyazaki 880-0027 (JP)**
 • **MATSUMOTO, Kunio**
  **Mino-shi, Osaka 562-0031 (JP)**

(74) Representative: **Kalhammer, Georg**
  **Lederer & Keller**
  **Patentanwälte**
  **Prinzregentenstrasse 16**
  **80538 München (DE)**

(54) **CELL-CONTAINING PREPARATIONS**

(57)    The present invention provides a cell-containing preparation comprising cells containing a DNA having a base sequence represented by SEQ ID NO:1 or 2, or a DNA hybridizable with a DNA having a base sequence represented by SEQ ID NO:1 or 2 under stringent conditions, and a fibrous protein.

   The cell-containing preparation of the present invention makes it possible to more efficiently supply NK4 having an inhibitory action against the growth or metastasis of cancers to cancer cells.

Fig. 1

**Description**

Technical Field

The present invention relates to a cell-containing preparation for providing NK4 to prevent and treat the growth, invasion and metastasis of cancers with use of an NK4-encoding DNA. In particular, the present invention provides a cell-containing preparation for preventing and treating the growth, invasion or metastasis of cancers by supplying NK4 secreted from cells transformed with an NK4-encoding DNA to cancer cells or cancer tissues, a method for inhibiting the growth, invasion or metastasis of cancers using the said preparation, and a method for producing the said preparation.

Background Art

Complete cure for cancers which readily undergo metastasis, such as pancreatic cancer, is difficult, and excision of organs at the affected part by cancers has been considered to be only an effective therapeutic method. However, since cancer cells which readily undergo metastasis rapidly invade into the surrounding tissues around the cancer cells, the metastatic cancer recurs with a quite high frequency when prognosis is insufficient after excision by surgery (see, for example, Takeda, "The role of adjuvant therapy for pancreatic cancer", Hepatogastroenterology, USA, 2001, Vol. 48, No. 40, p953-956; Cienfuegos JA, Analysis of intraoperative radiotherapy for pancreatic carcinoma, Eur J Surg Oncol., United Kingdom, 2000, Vol. 26-A, S13-15). Most of the causes of death by the cancer are not directly related to primary cancers, but are due to the recurred metastatic cancers.

Radiotherapies and chemotherapies such as administration of anticancer agents are usually used for the treatment of the cancer, and a therapeutic method by excision surgery of the tissues with an aid of the chemotherapy has been attempted in view of preventing the recurrence of cancers which readily undergo metastasis. Although inhibitory effects on cancer have been recognized in the radiotherapy and chemotherapy such as administration of anticancer agents, these therapeutic methods cannot be always considered to be safe, since they involve risks of various adverse effects such as myelosuppression, loss of hair, injuries of the heart and nerve, and injuries of the digestive tract and liver.

Accordingly, development of not only therapeutic methods of inhibiting proliferation of the primary cancer itself but also a method for preventing the onset of metastatic cancers have been urgently desired in the treatment of cancers. Furthermore, any therapeutic methods that satisfy both requirements without any adverse effects while being safe for the living body have not yet been established.

Hepatocyte growth factor (HGF) is a hetero-dimer protein composed of an α-chain and β-chain. It has been reported from many studies that HGF expresses a mitogenic activity, motogenic activity and morphogenic activity by binding to a c-Met/HGF receptor, and results in exhibiting actions of inducing invasion or metastasis of tumor cells, and angiogenesis (see , for example,

Kunio Matsumoto et. al, "HGF in lung morphogenesis and tumor infiltration: role as a mediator in epithelium-mesenchyme and tumor-stroma interactions", Cancer Chemother. Pharmacol., 1996, Vol. 38, Suppl, S42-7; Jiang. W.G et al, "HGF/scatter factor, its molecular, cellular and clinical implication in cancer", 1999, Crit. Rev. Oncol. Hematol, Vol. 29, p209-248).

NK4 is a peptide comprising an N-terminal hair-pin domain of the α-chain of HGF and four kringle domains, and acts as an antagonist of HGF by inhibiting the HGF from binding to the c-Met/HGF receptor (see, for example, Date K. et al., "Inhibition of tumor growth and invasion by four-kringle antagonist (HGF/NK4) for HGF", 1998, Oncogene, Vol. 17, No. 23, p3045-54; Parr C., et al., The HGF/SF-induced phosphorylation of paxillin, matrix adhesion, and invasion of prostate cancer cells were inhibited by NK4, an HGF/FS variant, 2001, Biochem. Biophys. Res Commun . , Vol 285 , No. 5 , p1330-7) . NK4 is known to inhibit growth, invasion or metastasis of tumors by its antagonist activity against HGF, and to inhibit the angiogenesis induced by HGF as well as by VEGF and bFGF through a mechanism different from that for the antagonist activity against HGF (see, for example, Kuba K., et al., "Kringle 1-4 of HGF inhibits proliferation and migration of human microvascular endothelial cells", 2000, Biochem. Biophys. Res. Commun., Vol. 279, No. 3, p846-52).

From the above facts, the growth and metastasis of cancers are inhibited by supplying NK4 to the cancer tissue . Furthermore, NK4 can be safely administered to the living body since it is a protein derived from the living body.

The methods for supplying NK4 to the cancer tissue include a method for administering NK4 itself, or a method using a gene therapy technology comprising directly introducing a NK4-encoding gene into cancer cells to produce NK4 therein. The method for directly introducing NK4 itself into the cancer cells is preferable for the reason that it can exhibit an immediate effect with an expectation of high therapeutic efficiency, however, the procedure of this method is complicated because it requires processes for cultivating a large amount of NK4-producing cells and for extracting and purifying NK4 from the cells.

On the other hand, the method for introducing the NK4-encoding gene into the cancer cells is advantageous in that NK4 is not required to be produced and purified in advance. Means generally used for introducing the gene into the cancer cells is to integrate the NK4-encoding gene into a vector such as an adenovirus vector, and to transfect the cancer cells with the vector.

The methods for introducing a gene encoding a protein effective for treating the cancer or the protein itself effective for treating the cancer into the cancer cells have been disclosed to date. These methods comprise transfecting the cancer cells with a vector by contact with the cancer tissue by covering the cancer tissue with light-curable gelatin in which the vector having the gene or the protein itself is contained, or allowing the protein to permeate into the cancer tissue (see, for example, Jain RK., et al., "Barriers to drug delivery in solid tumors", 1994, Sci Am., Vol. 271, No. 5, p498-504). While the protein was possible to be efficiently permeated into the cancer cell by the method above, it was difficult to effectively introduce the gene into the cancer cells since permeability of the vector as a large molecule into the cancer cells was low. While it may be more advantageous to supply the protein itself to the cancer cells from the above facts, this method cannot be always considered to be an excellent technology in view of its practical use because the protein should be independently produced and purified as described above.

Technologies that are able to practically and efficiently supply NK4 to the cancer cell such as the cell-containing preparation of the present invention have not been known in the past. In other words, the cell-containing preparation of the present invention is a technology involving both advantages of administering NK4 itself and of producing NK4 in cancer cells by introducing an NK4-encoding gene into the cancer cells directly. NK4 may be supplied to the cancer cells in the form of a protein having high permeability to the cancer cells without any necessity of independently producing and purifying NK4. Moreover, the preparation may be formed into a shape suitable for administration sites, for example the entire area of the cancer tissue as a target can be covered by forming the preparation into a sheet. Metastasis and recurrence of the cancer that has been left behind by surgery and is caused by the primary cancer may be also prevented by covering the remaining portions of the cancer after excision by surgery.

The cell-containing preparation of the present invention has no risks of onset of adverse effects as occur by using the chemotherapy as described above, and may be a safe anticancer agent or a cancer metastasis inhibitor for patients when the preparation is produced using patient's own cell samples from the patient.

Disclosure of the Invention

An object of the present invention is to provide a cell-containing preparation having an inhibitory effect for growth of cancers or metastasis and recurrence of cancers and having a safety to living bodies, in particular a cell-containing preparation capable of more efficiently supplying NK4 having an inhibitory action for growth or metastasis of cancers to cancer cells. Another object of the invention is to provide a method for inhibiting the growth or metastasis of cancers or for inhibiting the angiogenesis, using the cell-containing preparation. A still another object of the invention is to provide a method for producing the cell-containing preparation.

Cells containing a DNA having a base sequence represented by SEQ ID NO: 1 or 2, or a DNA hybridizable with a DNA having a base sequence represented by SEQ ID NO: 1 or 2 under stringent conditions are shaped with a fibrous protein, for example, collagen to form a complex, and this complex was transplanted around the cancer tissue. The inventors of the present invention have found, through intensive studies for attaining the above-mentioned objects, that NK4 produced in the cells in the form of complex can be more effectively supplied to the cancer tissue in proper quantities, and have completed the invention through additional studies based on these findings.

The present invention provides:

(1) A cell-containing preparation comprising a cell which has a DNA having a base sequence represented by SEQ ID NO: 1 or 2 or a DNA hybridizable with a DNA having a base sequence represented by SEQ ID NO: 1 or 2 under stringent conditions and a fibrous protein;

(2) the cell-containing preparation according to the above (1), wherein the cell is an epithelial cell of the oral mucosa, a skin cell or a fibroblast;

(3) the cell-containing preparation according to the above (1) or (2), wherein the fibrous protein is collagen;

(4) the cell-containing preparation according to any one of the above (1) to (3), wherein the cells are deposited on the surface of the fibrous protein;

(5) the cell-containing preparation according to any one of the above (1) to (4), wherein the cell is a transformant;

(6) the cell-containing preparation according to the above (5), wherein the transformant is transformed with a recombinant expression vector;

(7) the cell-containing preparation according to the above (6), wherein the recombinant expression vector is adeno-associated virus (AAV), retrovirus, poxvirus, herpes virus, herpes simplex virus, lentivirus (HIV), Sendai virus, Epstein-Barr virus (EBV), vaccinia virus, poliovirus, sindbis virus, SV40 or plasmid;

(8) the cell-containing preparation according to any one of the above (1) to (7) capable of forming a peptide encoded by a DNA having a base sequence represented by SEQ ID NO:1 or 2, or by a DNA hybridizable with a DNA having a base sequence represented by SEQ ID NO:1 or 2 under stringent conditions;

(9) the cell-containing preparation according to any one of the above (1) to (8) further containing a mesh sheet comprising a biodegradable resin;

(10) the cell-containing preparation according to the above (9), wherein the biodegradable resin is polyglycolic acid;

(11) the cell-containing preparation according to any one of the above (1) to (10), which is an anticancer agent or a cancer metastasis inhibitor;

(12) the cell-containing preparation according to the above (11), which is an anticancer agent or a metastasis inhibitor for ovarian cancer, pancreatic cancer, stomach cancer, gall bladder cancer, kidney cancer, prostate cancer, breast cancer, esophageal cancer, liver cancer, oral cavity cancer, colon cancer, large intestine cancer, sarcoma, glioma or melanoma;

(13) the cell-containing preparation according to any one of the above (1) to (10), which is an angiogenesis inhibitor;

(14) a method for inhibiting growth, invasion and metastasis of cancers or for inhibiting angiogenesis, which comprises administering the cell-containing preparation according to any one of the above (1) to (13) to a mammal;

(15) a method for producing a cell-containing preparation which comprises culturing a cell on the surface of a fibrous protein and transforming the cultured cells with a recombinant expression vector comprising a DNA having a base sequence represented by SEQ ID NO:1 or 2, or with a recombinant expression vector comprising a DNA hybridizable with a DNA having a base sequence represented by SEQ ID NO:1 or 2 under stringent conditions;

(16) a method for producing a cell-containing preparation, which comprises preparing a fibrous protein sheet by coating a fibrous protein onto a mesh sheet comprising a biodegradable resin; culturing a cell on the surface of the fibrous protein sheet obtained; and transforming the cultured cells with a recombinant expression vector comprising a DNA having a base sequence represented by SEQ ID NO:1 or 2, or with a recombinant expression vector comprising a DNA hybridizable with a DNA having a base sequence represented by SEQ ID NO:1 or 2 under stringent conditions; and

(17) a method for producing a cell-containing preparation, which comprises transforming the cells with a recombinant expression vector comprising a DNA having a base sequence represented by SEQ ID NO: 1 or 2, or with a recombinant expression vector comprising a DNA hybridizable with a DNA having a base sequence represented by SEQ ID NO: 1 or 2 under stringent conditions, and mixing the resulting transformant cells with a fibrous protein.

Brief Description of the Drawings

Fig. 1 is a schematic drawing showing an example of the cell-containing preparation of the present invention and a transplantation method thereof.

Fig. 2 is a schematic drawing showing recombinant adenovirus vector Ad-NK4.

Fig. 3 shows an example of an SEM analysis photograph (cross-section) of the cell-containing preparation of the present invention.

Fig. 4 is a graph obtained by monitoring time-dependent NK4 secretion of OMEC into which Ad-NK4 is introduced.

Fig. 5 shows an effect of introduction of Ad-NK4 on the growth of OMEC.

Fig. 6 is a graph showing an effect of NK4 secreted from into which Ad-NK4 is introduced, on inhibition of invasion of pancreatic cancer cells.

Fig. 7 is a graph showing concentrations of NK4 in the tumor and in the serum of mouse tumor model transplanted with an example of the cell-containing preparation.

Fig. 8 is a graph showing an effect for inhibiting the tumor volume from increasing in mouse tumor model transplanted with an example of the cell-containing preparation.

Fig. 9 is a graph showing an effect for inhibiting the number of angiogenesis from increasing in the mouse tumor model transplanted with an example of the cell-containing preparation.

In the drawings, the reference numeral 1 denotes collagen, the reference numeral 2 denotes VICRYL™ mesh sheet, the reference numeral 3 denotes OMEC, the reference numeral 4 denotes OMEC into which Ad-NK4 is introduced, the reference numeral 5 denotes a gene product (NK4), the reference numeral 6 denotes a cancer tissue, and reference numeral 7 denotes a tissue.

Best Mode for Carrying Out the Invention

The cell-containing preparation of the present invention comprises a cell having (a) DNA having a base sequence represented by SEQ ID NO: 1 or 2 or (b) a DNA hybridizable with a DNA having a base sequence represented by SEQ ID NO: 1 or 2 under stringent conditions, and a fibrous protein.

The base sequence represented by SEQ ID NO: 1 or 2 is an example of the NK4-encoding DNA. The bases from base No. 391 to base No. 405 in the base sequence represented by SEQ ID NO: 1 are deleted in the base sequence represented by SEQ ID NO: 2, and the protein produced by this DNA also has an antagonist activity against HGF and an inhibitory activity against angiogenesis.

The DNA hybridizable with a DNA having the base sequence represented by SEQ ID NO: 1 or 2 under stringent conditions in the present invention means, for example, a DNA obtained by using a colony hybridization method, plaque hybridization method or Southern hybridization method, using the above DNA as a probe. Specifically, the DNA may be identified by hybridization at about 65°C in the presence of about 0.7 to 1.0 M sodium chloride using a filter on which DNA derived from a colony or plaque is immobilized, followed by washing the filter at about 65°C using a 0.1 x to 2 x SSC solution (the composition of 1 x SSC solution comprises 150 mM sodium chloride and 15 mM sodium citrate).

Specific example of the DNA hybridizable with the DNA having the base sequence represented by SEQ ID NO: 1 or 2 is a DNA containing a base sequence having not less than 70%, preferably not less than 80%, more preferably not less than 90%, and most preferably not less than 95% of homology to the base sequence represented by SEQ ID NO:1 or 2. More specifically, the DNA sequence is partially modified by deleting, substituting or adding one or more bases in the base sequence represented by SEQ ID NO: 1 or 2 by various artificial processing, for example by site-specific mutagenesis, random mutation by treating with a mutagenesis agent or digestion with a restriction enzyme, so long as such modified DNA is able to produce a peptide having an antagonist activity against HGF and an inhibitory action against angiogenesis.

Hybridization may be performed by the known method, for example, by the method described in Molecular Cloning, A laboratory Manual, Third Edition (J. Sambrook et al., Cold Spring Harbor Lab. Press, 2001: abbreviated as Molecular Cloning, Third Edition) . When commercially available libraries are used, the method may follow the technical instruction attached.

Specific examples of the DNA having the base sequence in which one or more bases are deleted, substituted or added in the base sequence represented by SEQ ID NO:1 or 2 is (a) a base sequence in which one or two or more (preferably about 1 to 30, more preferably about 1 to 10, and more preferably several (about 1 to 5)) bases are deleted in the base sequence represented by SEQ ID NO:1 or 2, (b) a base sequence in which one or two or more (preferably about 1 to 30, more preferably about 1 to 10, and more preferably several (1 to 5) bases are added in the base sequence represented by SEQ ID NO: 1 or 2, (c) a base sequence in which one or two or more (preferably about 1 to 30, more preferably about 1 to 10, and more preferably about 1 to 5) bases are substituted with other bases in the base sequence represented by SEQ ID NO:1 or 2, or (d) a base sequence having a base sequence in combination thereof.

Substitution or other operations may be applied by the known method such as ODA-LA PCR method, gapped duplex method or Kunkel method, or a method similar to these methods using PCR and known kits, for example Mutan™-superExpress Km (Takara Shuzo Co.) or Mutan™-K (Takara Shuzo Co.).

For cloning a DNA fragment having a complete base sequence of (a) the DNA having the base sequence represented by SEQ ID NO: 1 or 2 or (b) a DNA hybridizable with the DNA having the base sequence represented by SEQ ID NO:1 or 2 under stringent conditions, the fragment is amplified by a PCR method using a synthetic DNA primer having a partial base sequence of (a) or (b), for example, a known HGF primer (for example a DNA fragment having a base sequence represented by SEQ ID NO:5 or 6); or the DNA may be selected by hybridization from DNAs in which appropriate vectors are integrated using a DNA fragment or a synthetic DNA encoding a partial or whole sequence of labeled HGF protein.

The DNA may be cloned by chemical synthesis using known methods from known base sequence information of HGF. An example of the chemical synthesis method is to use a DNA synthesizer such as DNA synthesizer model 392 (manufactured by Perkin-Elmer Co.) by the phosphoramidite method.

The DNA used in the present invention may be modified for enhancing its stability in the cells or for reducing its toxicity, if any. Many modification methods are known in the art, and may be carried out according to the method disclosed in Kawakami et al., Pharm Tech Japan, Vol.8, pp.247, 1992; Vol.8, pp.395, 1992; S. T. Crooke et al. ed., Antisense Research and Applications, CRC Press, 1993.

The 3'-end or 5'-end of the DNA used in the present invention may be chemically modified with a protective group known in the art, such as polyethylene glycol and tetraethylene glycol in order to prevent decomposition with nucleases such as exonuclease and RNase.

The DNA may have ATG as a translation initiation codon at the 5'-end and TAA, TAG or TGA as a translation stop codon at the 3'-end. These translation initiation codons and translation stop codons may be added to the DNA using an appropriate DNA adapter. It is preferable that the DNA has a polyadenyl sequence at the 5'-end.

An NK4-encoding RNA may be used in the present invention so long as it is able to express an antagonist activity against HGF and an angiogenesis inhibitory activity by treatment with a reverse transcriptase, and the RNA may be obtained by known methods. Such RNA is to be also included in the DNA used in the present invention.

The cell used in the present invention comprises (a) a DNA having a DNA sequence represented by SEQ ID NO:1 or 2, or (b) a DNA hybridizable with the DNA having a DNA sequence represented by SEQ ID N0:1 or 2 under stringent conditions.

The method for introducing the above DNA (a) or (b) into the cells used in the present invention is not particularly restricted, and known methods may be used. For example, while such method include a method of introducing the DNA into the cells by allowing it to be contained in recombinant expression vectors such as plasmids and viruses, or in artificial vectors such as liposomes and microcapsules, a recombinant expression vector is preferably used in the present invention. Any methods known per se may be used for introducing the recombinant expression vector into a host. Examples of such methods include a competent cell method [J. Mol. Biol., 53, 154 (1970)], DEAE dextran method [Science, 215, 166 (1982)], in vitro packaging method [Proc. Natl. Acad. Sci., USA, 72, 581 (1975)], virus vector method [Cell, 37, 1053 (1984)], micro-injection method [Exp. Cell. Res., 153, 347 (1984)], electroporation method [Cytotechnology, 3, 133 (1990)], calcium phosphate method [Science, 221, 551 (1983)], and lipofection method [Proc. Natl. Acad. Sci., USA, 84, 7413 (1987)], and protoplast method [Japanese Patent Application Laid-Open No. 63-2483942, Gene, 17, 107 (1982), Molecular & General Genetics, 168, 111 (1979)].

The recombinant expression vector used in the present invention is preferably an expression vector capable of effectively producing NK4 by allowing the DNA of (a) or (b) to be expressed in the cell into which the expression vector is introduced. For example, a DNA fragment containing the base sequence encoding NK4 is cut from cDNA, and the DNA fragment is linked to the downstream of the promoter in an appropriate expression vector to produce the recombinant expression vector.

Examples of the expression vector available include plasmids derived from Escherichia coli such as pCR4, pCR2.1, pBR322, pBR325, pUC12 and pUC13; plasmids derived from Bacillus subtilis such as pUB110, pTP5 and pC194; plasmids derived from yeast such as pSH19 and pSH15; bacteriophages such as λ phage; and animal viruses such as adenovirus, adeno-associated virus (AAV), retrovirus, poxvirus, herpes virus, herpes simplex virus, lentivirus (HIV), Sendai virus, Epstein-Barr virus (EBV), vaccinia virus, polio virus, sindbis virus and SV40; as well as pA1-11, pXT1, pRc/CMV, pRc/RSV and pcDNAI/Neo. Viruses are preferable among them as the vector used in the present invention, and retrovirus, adeno-associated virus and adenovirus are more preferable.

While adenovirus belongs to various serotypes, human adenovirus types 2 or 5 are preferably used in the present invention. Use of the adenovirus vector is further preferable because adenovirus is known to have higher transfection efficiency as compared with other viruses, to be able to infect undivided cells, and not to be integrated into genomes of cells.

The virus vector is preferably a replication-defective virus in which virus genes are completely or almost completely deleted. At least the E1 region of the adenovirus vector is preferably non-functional. Other regions may also be modified, and any regions of E3 region (WO 95/02697), E2 region (WO 94/28938), E4 region (WO 94/28152, WO 94/12649, WO 95/02697) or late genes L1 to L5 may be particularly modified. Modified viruses such as replication-defective viruses may be produced by the method known per se. The modified viruses may also be recovered and purified by the method known per se. The modified viruses available are described, for example, in Japanese Patent Application National Publication Laid-Open Nos. 11-514866, 11-506311, 9-500524, 8-501703 and 8-508648, or Japanese Patent Application Laid-Open No. 8-308575.

The promoter may be any one of appropriate promoters corresponding to a host used for expressing the gene. Forexample, when a mammal is used as a host, examples of the promoter include those obtained from the genome of viruses such as Rous sarcoma virus (RSV virus), MPSV, polyoma virus, fowlpox virus, adenovirus, bovine papilloma virus, avian sarcoma virus, cytomegalovirus (SMV), hepatitis B virus, simian virus (SV40) and vaccinia virus , and metallothionein promoter and heat shock promoter.

A vector to which the following regulatory sequence is added may be used for the recombinant expression vector in order to allow the DNA encoding NK4 to be expressed, or in order to be advantageous for expression. Each regulatory sequence may be either endogenous or exogenous to the vector.

While examples of the regulatory sequence include a signal sequence, promoter, pro-peptide sequence, enhancer, selection marker and terminator, it is not restricted thereto. The regulatory sequence may have a linker in advance so as to facilitate a linkage between the DNA encoding NK4 and the regulatory sequence, and/or a linkage between the regulatory sequences.

NK4 produced in the host cell is actively secreted out of the host cell by integrating the signal sequence into the recombinant expression vector. Consequently, NK4 produced may be efficiently supplied to a desired site to inhibit the growth and metastasis of the cancer, and angiogenesis.

While an HGF signal sequence, an insulin signal sequence and an α-interferon signal sequence may be used as the signal sequence when the host is mammal cells, the HGF signal sequence derived from human is preferably used in particularly in the present invention.

The methods known per se may be used for adding the signal sequence, and examples of such method include those described in J. Biol. Chem. , 264, 17619 (1989), Proc. Natl. Acad. Sci., USA, 86, 8227 (1989), Genes Develop., 4, 1288 (1990), Japanese Patent Application Laid-Open No. 5-336963, and WO 94/23021.

An enhancer is preferably introduced into the vector when the cells of higher mammals, for example, human cells are used as the host. Introducing the enhancer permits transcription of the DNA inserted into the vector to be increased. Examples of the enhancer include an SV40 enhancer, a cytomegalovirus enhancer, an initial promoter/enhancer of cytomegalovirus, a polyoma enhancer and an adenovirus enhancer.

Examples of the selection marker include dihydrofolic acid reductase gene (methotrexate (MTX) resistant), ampicillin resistant gene and neomycin resistant gene. In particular, the desired gene may be selected in a culture medium not containing thymidine when the DHFR gene is used as the selection marker using CHO (DHFR-) cells.

The cells constituting the cell-containing preparation of the present invention capable of producing NK4 may be produced by inserting an NK4-encoding DNA fragment into these virus vectors and transfecting the host cells with the DNA fragment to transform the cells.

The method for preparing the virus vector, and the method for inserting the DNA fragment into the virus vector are described in Experimental Medicine, Supplement Edition, Basic Technology of Gene Therapy, Yodo-Sha Co. (1996), and in Experimental Medicine, Supplement Edition, Experimental Methods of Gene Introduction & Expression Analysis, Yodo-Sha Co. (1997).

While the cells used for the cell-containing preparation of the present invention are not particularly restricted so long as they can serve as host cells for the recombinant expression vector, examples of such cells include animal cells, cells of the genus Escherichia, cells of the genus Bacillus, Lactobacillus bifidus, lactic acid bacteria, yeast, and insect cells.

Examples of the animal cells available include epithelial cells of oral cavity mucous membrane (hereinafter abbreviated as OMEC), skin cells, fibroblasts, and somatic cells including various epithelial cells of mammals such as human, or simian COS-7 cells, Vero, Chinese hamster CHO cells (hereinafter abbreviated as CHO cells), dhfr gene-deficient Chinese hamster cells (abbreviated as CHO (dhfr-) cells), mouse BALB/3T3 cells, mouse L cells, mouse AtT-20 cells, mouse C127 cells, mouse myeloma cells, rat GH3 cells, human HeLa cells, human FL cells, 293 cells derived from the human fetal kidney (Jikken Igaku (Experimental Medicine), 12, 316, (1994), and various cell strains including mouse NIH3T3 cells.

The animal cells may be any one of established cells in the laboratory and cells isolated from living tissues. The cells extracted from the tissue can be obtained by treating the tissue extracted by an appropriate means with Dispase or EDTA, followed by treating with trypsin into single cells. The single cells obtained are then cultured to confluence in an appropriate culture medium, and a cell line is established by repeating passage culture twice or three times. The established cells are sampled as single cells again by treating with trypsin and collagenase, and are used as the cells of the present invention. The feeder layer method may be used depending on the kinds of the cells. For example, fibroblasts derived from mouse fetus (3T3 cells) are preferably used as feeder cells when MEC or keratinized epidermal cells are used as the cells of the present invention. The feeder layer method may be used according to the known methods described, for example, in Ueda M, The potential of Oral mucosal cells for cultured epithelium: a preliminary report. Ann. Plast. Surg., 35 (5), p498-504 (1995), Rheinwald JG, Serial cultivation of strains of human epidermal kerationocytes: the formation of keratinizing colonies from single cells., Cell., 6 (3), p331-343 (1975).

Specific examples of the genus Escherichia available include Escherichia coli K12/DH1 [Proc. Natl. Acad. Sci. USA, Vol. 60,160 (1968)]; JM103 [Nucleic Acids Research, Vol 9, 309 (1981)]; JA221 [Journal of Molecular Biology, Vol. 12, p517 (1978)]: HB101 [Journal of Molecular Biology, Vol. 41, p459 (1969)]; C600 [Genetics, Vo. 39, 440 (1954)], DH5$\alpha$ [Inoue, H., Nojima, H. and Okayama, H. , Gene, 96, p23-28 (1990)], and DH10B [Proc. Natl. Acad. Sci. USA, Vol. 87, p4645-4649 (1990)]. Examples of the genus Bacillus available include Bacillus subtilis MI114 [Gene, Vol. 24, 255 (1983); Journal of Biochemistry, Vol. 95, 87 (1984)]. Examples of the genus Bifidus include Bifidobacterium longum, Bifidobacterium bifidum, and Bifidobacterium breve. Examples of lactic acid bacteria include the genus Lactobacillus, Streptoccoccus, Leuconostoc and Pediococcus.

Examples of yeast available include Saccharomyces cerevisiae AH22, AH22R-, NA87-11A, DKD-5D, 20B-12, Schizosaccharomyces pombe NCYC1913 and NCYC2036, and Pichia pastoris.

When the virus is AcNVP, specific examples of insect cells available include spodoptera frugiperda cell line (Sf cell) derived from larva, MG1 cells derived frommidgut of Trichoplusia ni, High Five™ cells derived from eovum of Trichoplusia ni, cells derived from Mamestrabrassicae and cells derived from Estigmena acrea. When the virus is BmNPV, cells derived from Bombyx mori N (BmN cell) are used. Examples of the Sf cells available include Sf9 cell (ATCC CRL1711), Sf21 cell (Vaughn, J.L. et al., [In Vivo, 13, 213-217, (1977)].

DNA may be introduced according to the method described in Saibou Kohgaku (Cell Technology), Supplement Edition 8, Sin Saibou Kougaku Jikken Purotokohru (Protocol of New Experimental Cell Technology), 263-267 (1995), published by Shujun-sha Co, and Virology, Vo. 52, p456 (1973).

DNA can be introduced into the genus Escherichia, for example, by the method according to Proc. Natl. Acad. Sci. USA, Vol. 69, 2110 (1972) and Gene, Vol. 17, 107 (1982). The genus Bacillus may be transformed, for example, by the method according to Molecular & General Genetics, Vo. 168, 111 (1979).

DNA can be introduced into yeast, for example, by the methods described in Methods in Enzymology, Vol. 194, p182-187 (1991) and Proc. Natl. Acad. Sci. USA, Vol. 75, p1929 (1978).

DNA may be introduced into insect cells by the method, for example, described in Bio/Technology, Vol. 6, p47-55 (1988).

Transformants into which an expression vector containing an NK4-encoding DNA is introduced can be thus obtained.

The preparation of the present invention comprises a cell containing an NK4-encoding DNA as well as a fibrous protein. The fibrous protein serves as a carrier of the cells containing the NK4-encoding DNA in the cell-containing preparation of the invention. Containing the fibrous protein permits the preparation of the invention to be formed into various shapes such as a sheet, sphere and tube, and the preparation may be administered to all sites in the body. Examples of the fibrous protein include collagen, keratin, fibroin, elastin and fibrin. Collagen, especially collagen type I as a major constituting element of the dermis and cartilage tissues of mammals is preferable in the present invention. The fibrous protein is safe to the living body since it is a protein derived from organisms such as mammals.

It is preferable for the fibrous protein used in the present invention to contain components of a culture medium suitable for culturing the cells of the invention. By including culture medium components in the fibrous protein, nutrients can be supplied to the cells in the preparation of the present invention, leading to stable and longer survival of the cells when the preparation of the present invention is administered to the living body. As a result, the NK4 supply to the cancer tissue can be stabilized and extended for a longer period of time.

A collagen gel containing the culture medium component can be prepared by the steps comprising: mixing the culture medium component suitable for culturing the cells used in the present invention with the fibrous protein, for example collagen type I; allowing the mixture to stand at 30°C to 37°C for 15 minutes to 1 hour; and solidifying the mixture into a desired shape, for example, a sheet, sphere or tube. When the collagen gel containing the culture medium component is formed into a thick shape, for example, into a sheet, the collagen gel containing the culture medium component may be fixed on a mesh sheet comprising a biodegradable resin to prepare a collagen gel composite material in order to maintain its strength and to simplify its handling. Although the method for preparing the collagen gel composite material is not particularly restricted, the collagen gel may be prepared on the mesh sheet by pouring a mixture of collagen and culture medium component onto the mesh sheet comprising the biodegradable resin.

A mesh sheet of a surgical suture made of a biodegradable polymer material is preferable for the mesh sheet of the biodegradable resin. Particularly, polyglycolic acid is preferable as the biodegradable polymer in the present invention, and an example of the mesh sheet of the biodegradable resin is knitted-type VICRYL$^{TM}$ mesh; Ethicon, Inc., New Jersey.

While the cell-containing preparation of the present invention may contain other additives such as delayed-release agents, isotonizing agents and pH control agents that are substantially admitted to add to the conventional pharmaceutical preparations under the Pharmaceutical Affairs Law, it is preferable that the kind and content of them do not interfere with proliferation, survival and/or NK4 secretion of the cells contained in the preparation of the present invention, and the content thereof is preferably within a range not impairing the object of the invention.

These other additives may be incorporated into the culture medium in advance when the collagen gel is prepared. Proteins such as gelatin, porous ceramics, polyamino acids, polylactic acid, chitin or chitin derivatives, and water-swellable polymer gel may be used for the delayed-release agent. The isotonizing agent available includes polyethylene glycol and sodium chloride, and the pH control agent includes phosphate salts and amino acids.

The collagen material containing the culture medium components may be further compacted so as to have a high density, if necessary, for enhancing the strength thereof. Although there is no particular limitation to the compacting method, a pressure is applied from above the collagen gel which is solidified using a medical tool such as a rubber spatula or rubber cup, for example, silicone rubber cup.

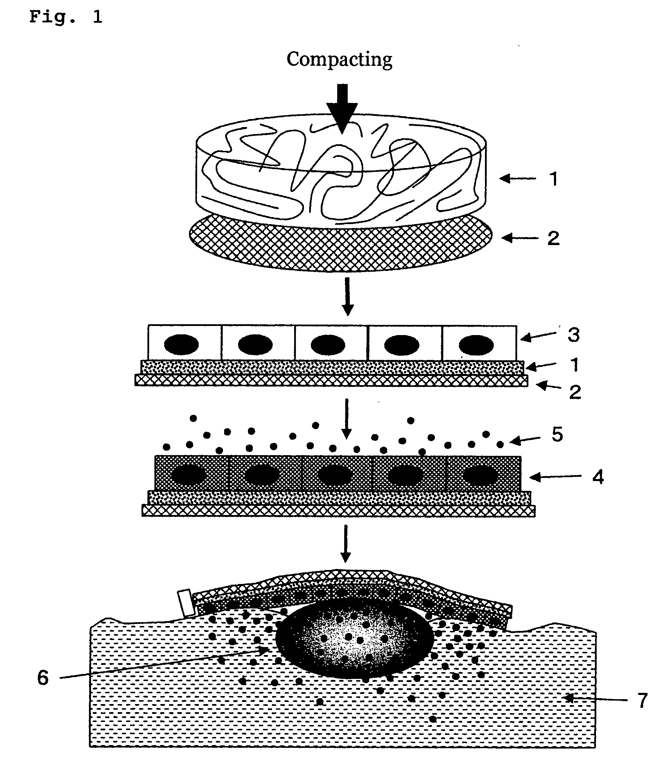

While the preparation of the present invention is characterized by comprising the fibrous protein containing the culture medium components as prepared above, for example, the collagen gel containing the culture medium components, and the cells containing an NK4-encoding DNA , the collagen gel containing the culture medium components may be either integrated or not integrated with the cells containing the NK4-encoding DNA. The cells are dispersed in the collagen gel in the integrated preparation, and such cell-dispersing collagen gel may be prepared by mixing the cells in the culture medium components in advance when the collagen gel is prepared. The non-integrated preparation is defined to be other than the integrated preparation, for example, the cells are laminated on the surface of the collagen gel. Such cell-laminated collagen gel may be prepared by applying or seeding the cells on the surface of the collagen gel followed by culturing the cell after the collagen gel is prepared. Preferably, the cells and the collagen gel are not integrated with each other, or the cells are particularly preferably laminated on the surface of the collagen gel. The cell layer may be a monolayer or multilayer. Such layered structure permits the cell-containing layer to directly contact the administration site so that NK4 secreted from the cells can be efficiently supplied to the cancer cells (see Fig. 1).

The NK4-encoding DNA may be introduced into the cells used in the present invention before being integrated or not integrated with the collagen gel. Otherwise, the cells may be converted into the cells containing the NK4-encoding DNA through introduction of the NK4-encoding DNA after the cells are integrated or not integrated with the collagen gel.

When the cells are integrated with the collagen gel in the present invention, a DNA is preferably introduced into the cell in advance considering introduction efficiency of DNA into the host cell. On the other hand, when the cells are not integrated with the gel as a preferable embodiment of the present invention, i.e. when the cells are laminated on the surface of the collagen gel, it is preferable to introduce the NK4-encoding DNA into the laminated host cells, which are cultured on the collagen gel, using a vector such as a virus vector.

Although the culture medium contained in the fibrous protein material is not particularly limited so long as it is a culture medium usually used for cultivation of animal cells when the host cell is an animal cell, examples of such medium available include an MEM medium [Science, Vol., 501 (1952)] containing about 5 to 20% of fetal bovine serum (FBS), DMEM medium [Virology, Vol. 8, 396 (1959)], RPMI 1640 medium [The Journal of the American Medical Association, Vol. 199, 519 (1967)], and 199 medium [Proceeding of the Society for the Biological Medicine, Vol. 73, 1 (1950)]. The pH for cultivating the cell is preferably about 6 to 8. The cells are usually cultivated at 30 to 40°C for about 15 to 60 hours, and are aerated and stirred, if necessary. The culture conditions such as the temperature, oxygen concentration and carbon dioxide concentration may be appropriately determined depending on the cells used.

While the culture medium used for cultivation is not particularly restricted so long as it contains carbon sources, nitrogen sources and inorganic salts necessary for the growth when the host is the genus Escherichia or the genus Bacillus, preferable examples thereof includes LB medium (manufactured by Nissui Pharmaceutical Co.), and M9 medium containing glucose and casamino acid (Miller, Journal of Experiments in Molecular Genetics, 431-433, Cold Spring Harbor Laboratory, New York, 1972). If necessary, chemicals such as 3β-indolylacrylic acid may be added in order to permit the promoter of the vector containing the NK4-encoding DNA to efficiently work. When the host is the genus Escherichia, the cells are usually cultured at 15 to 43°C for about 3 to 24 hours with optional stirring and aeration. When the host is the genus Bacillus, the cells are usually cultured at 30 to 40°C for about 6 to 24 hours with optional stirring and aeration. When the host is yeast, examples of the culture medium include Baekholder minimum medium [Bostian, K. L. et al., Proc. Natl. Acad. Sci. USA, Vol. 77, p4505 (1980)], and SD medium containing 0.5% casamino acid [Bitter, G. A. et al., Proc. Natl. Acad. Sci. USA, Vol. 81, p5330 (1984)]. The pH of the culture medium is preferably adjusted to about 5 to 8. Cultivation is usually continued for about 24 to 72 hours at about 20 to 35°C with aeration and stirring, if necessary.

When the host is an insect cell, the culture medium used is Grace's Insect Medium [T.C.C., Nature, Vol. 195, 788 (1962)] in which an additive such as 10% of inactivated bovine serum is appropriately added. The pH of the medium is preferably controlled to about 6.2 to 6.4. The cells are cultured at about 27°C for about 3 to 5 days with optional aeration and stirring.

The cell-containing preparation of the present invention is characterized in that NK4 is produced through incorporation of NK4-encoding DNA or RNA into the host cell. NK4 produced by the cell-containing preparation of the present invention is a peptide encoded by (a) a DNA comprising a base sequence represented by SEQ ID NO:1 or 2, or encoded by (b) a DNA hybridizable with a DNA comprising the base sequence represented by SEQ ID NO:1 or 2 under stringent conditions, and exhibits an antagonist activity against HGF and an inhibitory action against angiogenesis. Examples of the particularly preferable protein include (c) a peptide comprising an amino acid sequence represented by SEQ ID NO: 3 or 4, or (d) a peptide substantially identical to the peptide comprising the amino acid sequence represented by SEQ ID NO: 3 or 4 and having an amino acid sequence in which one to several amino acids are deleted, added or substituted, wherein the peptide has an antagonist activity against HGF and an inhibitory action against angiogenesis.

The basic operation of the gene engineering and bioengineering in the present invention are described in commercially available experimental textbooks by Idenshi Manyuaru (Gene Manipulation Manual), Kodansha Ltd.; Yasutaka Takagi, ed., Idenshi Jikken Sohsa Hou (Method of Gene Manipultion), Kodansha Ltd.; Molecular Cloning Vol. 3, Methods in Enzymol.), 194 (1991); and Jikkenn Igaku (Experimental Medicine), supplement edition, Method of Gene Experiment using Yeast, Yohdo-sha Co. (1994).

The cell-containing preparation of the present invention can be formed into various shapes such as a sheet, sphere or tube suitable for administering to various sites by adding a fibrous protein such as collagen as a carrier, and is able to administer by embedding into subcutaneous and intramuscular sites. For example, the cancer tissue itself may be covered with the sheet by forming the preparation into a sheet to enable NK4 to supply to respective cells in the entire cancer tissue in proper quantities. Metastasis and recurrence of the cancer after the surgery may be prevented by applying the sheet so as to cover the site where the cancer tissue has been removed through a surgical operation.

Although the dosage of the cell-containing preparation of the present invention may be controlled depending on the kind of diseases to be cured, condition, age and bodyweight of patients, and administration sites, it is usually 0.01 to 2000 mg, preferably 0.1 to 100 mg, as the weight of the NK4-encoding DNA for a patient with a body weight of 60 kg. The preparation may be administered to other animals by converting the dosage per 60 kg of the body weight.

The cell-containing preparation of the present invention can be used as preventive and therapeutic agents of diseases caused by cancers and/or angiogenesis, and as inhibitors for cancer metastasis. Accordingly, the cell-containing preparation of the present invention may be used for inhibition of invasion, growth and metastasis of tumors, induction of apoptosis, and/or inhibition of angiogenesis. The present invention also provides a method for inhibiting invasion, growth and metastasis of tumors, induction apoptosis and/or inhibition of angiogenesis using the cell-containing preparation of the present invention.

Examples of the cancer as the target disease of the present invention include lung cancer, ovarian cancer, pancreatic cancer, stomach cancer, gall bladder cancer, kidney cancer, prostate cancer, breast cancer, esophageal cancer, liver cancer, oral cavity cancer, colon cancer, large intestine cancer, uterine cancer, bile duct cancer, islet cell cancer, adrenal cortex cancer, bladder cancer, testicular cancer, testicular tumor, thyroid cancer, skin cancer, malignant carcinoid tumor, malignant melanoma, osteosarcoma, soft tissue sarcoma, neuroblastoma, Wilms' tumor, retinoblastoma, melanoma and glioma. Among them, ovarian cancer, pancreatic cancer, stomach cancer, gall bladder cancer, kidney cancer, prostate cancer, breast cancer, esophageal cancer, liver cancer, oral cavity cancer, colon cancer, large intestine cancer, sarcoma, melanoma and glioma are preferable , and ovarian cancer, pancreatic cancer, stomach cancer and gall bladder cancer are particularly preferable.

Examples of diseases due to angiogenesis include rheumatoid arthritis, psoriasis, Osler-Webber syndrome, myocardial vasculogenesis, telangiectasia, hemophilic arthritis, ocular vasculogenesis (for example, diabetic retinopathy, retinopathy of prematurity, age-related macular degeneration, corneal transplant rejection, neovascular glaucoma, retrolental fibroplasia, or perosis), vascular fibroma, benign tumor (for example, hemangioma, acoustic neuroma, neurofibroma, trachoma, pyogenic granuloma), tumor of hemopoietic organ including leukemia,solid tumor,tumor metastasis, granulation wound, and the like. The cell-containing preparation of the present invention may be used in combination with surgical treatment, radiotherapy and chemotherapy. Excision of tumors is one of the surgical treatment. Radiotherapy include γ-ray, X-ray, microwaves or electron beam irradiation. Chemotherapy includes administration of antitumor agents. Examples of the antitumor agent include alkylation agents, various metabolic antagonists, antitumor antibiotics, antitumor plant extracts and BRM (biological response modulators).

The effect of the cell-containing preparation of the present invention as anticancer agents and inhibitors of cancer metastasis, i.e. antagonist activity against HGF and inhibitory action against angiogenesis involved in the preparation of the present invention may be determined according to the known methods, for example, according to the determination method to be described hereinafter.

Examples

While examples of the invention are described hereinafter, the disclosure below represents only preferable examples, and does not restrict the technical scope of the present invention in any sense.

(Production Example) Preparation of cell-containing preparation

(1) Preparation of NK4 cDNA

mRNA was isolated from subcutaneous tissue cells of Wister rat or OMEC using ISOGEN-LS (Nippon Gene Co. , Ltd., Toyama, Japan), and the mRNA was used for RT-PCR(reverse transcription/polymerase chain reaction) to isolate NK4 cDNA. Specifically, 0.5 μl of mRNA solution (150 ng of mRNA), and 5 μl of 10×RT-PCR solution (500 mM KCl, 100 mM Tris-HCl (pH 9.0), 1% Triton X-100 , 15 mM MgCl$_2$), 4 μl of dNTP (2.5 mM), 2 μl of primer 1 (10 mM), 2 μl of primer 2 (10 mM), 0. 5 μl of Taq polymerase (Takara), 0.5 μl of RNasin (Promega), 0.5 μl of reverse transcriptase (Takara) and 35.2 μl of DEPC-treated H$_2$O were mixed. The reverse transcription reaction was performed at 42°C for 30 minutes and at 95°C for 5 minuets, and a cycle of 94°C for 30 seconds, 55°C for 1 minute and 72°C for 1 minute was repeated 40 times, followed by a reaction at 72°C for 7 minutes to obtain NK4 cDNA. NK4 cDNA thus obtained was cloned into pCRII™ vector using TA Cloning Kit (Invitrogen) to obtain pCRII/NK4. The primer used was the DNA fragment represented by SEQ ID NO: 5 or 6.

(2) Construction of recombinant expression vector

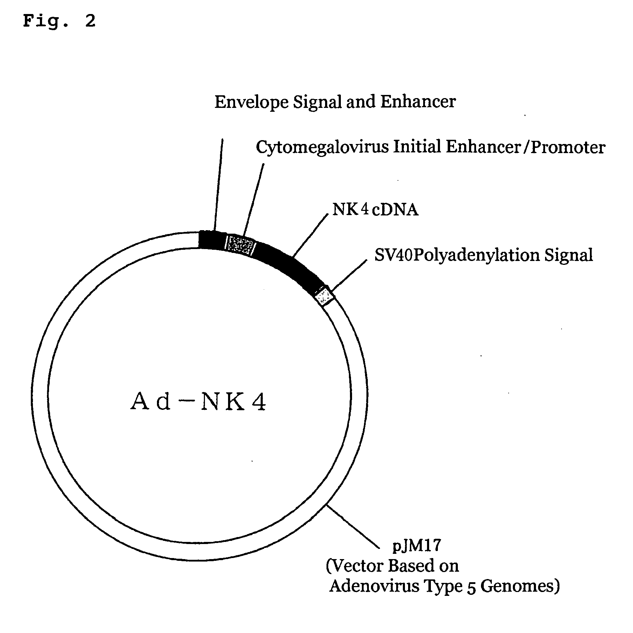

NK4 cDNA integrated into pCRII vector prepared in the above (1) was cut with a restriction enzyme KpnI/SpeI, and the cut-end was blunted by treating with T4 DNA polymerase (Takara). The NK4 cDNA fragment obtained was treated with a restriction enzyme XhoI, mixed with human adenovirus vector V (E1a deficient, E3 partially deficient) having a cut-end that has been blunted, and an NK4 expression vector Ad-NK4 was obtained by ligating the fragment with the vector using T4 DNA ligase (Fig. 2).

(3) Establishment of OMEC

Intraoral tissues were sampled from Wister rat with age 3 to 6 weeks and were subdivided. The tissue pieces were immersed twice in PBS (pH7.4.4, Nissui Pharmaceutical Co., Ltd., Tokyo, Japan) containing antibiotics (1000 U/ml of penicillin G potassium, 1 mg/ml of kanamycine and 2.5 μg/ml of amphotericin B). The tissue after immersion was immersed again in a DMEM culture medium (Gibco Laboratories Inc., Grand Island, NY) containing 0.2% dispase (Sigma-Aldrich Co., St. Louis, MO). Subsequently, the tissue was treated at room temperature for 30 minutes using a solution containing 0.25% of trypsin and 5mM EDTA, followed by washing with a DMEM culture medium containing 10% of FBS (CSL Ltd. , Victoria, Australia). The sample tissue obtained was stirred for 30 minutes in the DMEM culture medium containing 5% of FBS to release the cells, and the free OMEC cells were obtained by filtering with a filter with a pore size of 50 μm.

After treating Swiss 3T3 cells (Dainippon Pharmaceutical Co., Ltd.; Osaka, Japan) with 4 μg/ml of mitomycin C (Wako Pure Chemical Industries, Tokyo, Japan) for 2 hours, $1 \times 10^5$ cells were seeded on each well (Costar Inc., NY) of 6-well plate filled with EFM culture medium (DMEM culture medium: Ham's F culture medium (Nihonseiyaku, Tokyo, Japan)= 3 :1) containing 10% $CO_2$. The 10% $CO_2$-containing EFM culture medium was supplemented with 5% FBS, 5 μg/ml insulin (Wako Pure Chemical Industries), 5 μg/ml transferrin (Wako Pure Chemical Industries), $2 \times 10^{-9}$ M triiodo-tyrosine (Sigma-Aldrich Co.), 10 ng/ml cholera toxin (Sigma-Aldrich Co.), 0.5 μg/ml hydrocortisone (Wako Pure Chemical Industries), 100 U/ml penicillin, 0.1 mg/ml kanamycine and 0.25 mg/ml of amphotericin B.

Subsequently, $1 \times 10^5$ of free OMEC was seeded on each well. On day three after seeding the cell, 10 ng/ml of epidermal growth factor (human recombinant epidermal growth factor: Wako Pure Chemical Industries) was added to each well. After confirming that OMEC had grown to confluence after 7 to 10 days, the cells were subjected to passage culture. The second or third passage culture cells were harvested as established cells.

(4) Preparation of collagen gel

Knitted mesh sheet VICRYL™ (polyglactin 910; Nithicon, Inc., New Jersey) was placed on each well of a 24-well plate (Greiner Bio-one Co. , Ltd. , Frickenhausen, Germany), and a mixed solution of 0.5 ml of 10% FBS-containing DEM culture medium and 0.5 ml of collagen type I solution (0.3% by mass; CELLGEN, Koken Corp. , Tokyo, Japan) suspended in the same culture medium was poured onto the mesh sheet. A collagen-VICRYL™ mesh composite material was prepared by allowing the mesh sheet to stand at 37°C for 30 minutes. The collagen gel on the composite material was compacted by compression using a silicone cup.

(5) Preparation of collagen gel-adhered OMEC sheet

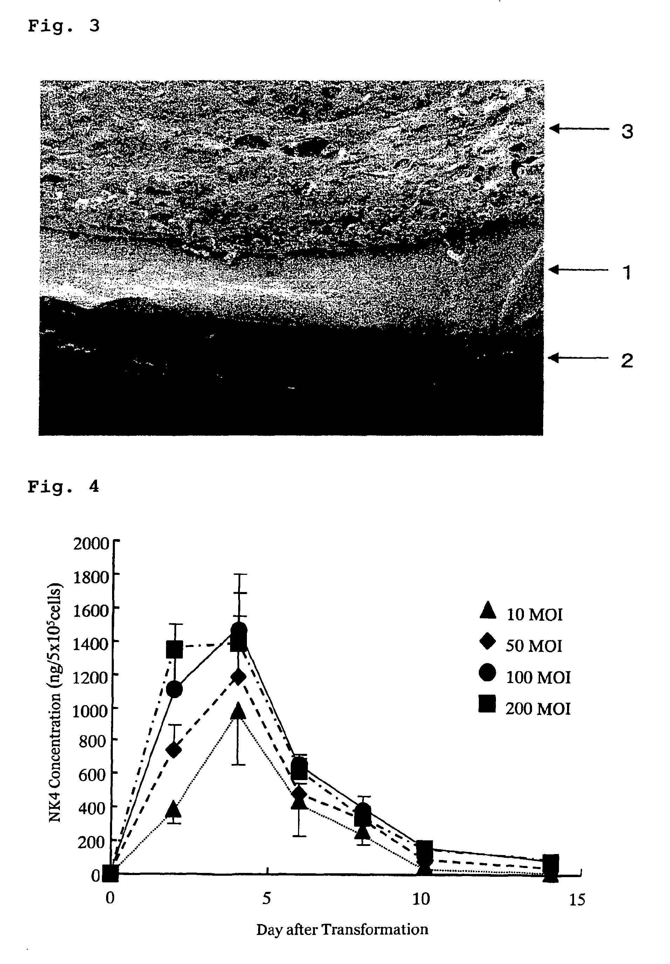

Established OMEC obtained in the above (3) was treated to free cells using 0.05% trypsin-EDTA. The free cells obtained were seeded on the surface of the collagen gel obtained in the above (4) in a density of $2 \times 10^5$ cells/cm$^2$. An OMEC layer was formed on the surface of the collagen gel by cultivation at 37°C for 2 to 3 days. Fig. 3 shows a cross section of the collagen gel-adhered OMEC sheet photographed by SEM (scanning electron microscope). It was confirmed from Fig. 3 that the collagen gel-adhered OMEC sheet comprises three layers of an OMEC cell layer, a collagen gel layer and a VICRYL™ mesh sheet. An electron microscope JSM-840A manufactured by JOEL Co. was used for the SEM analysis.

(6) Introduction of Ad-NK4 into OMEC

Five hundreds μl of 2% FBS-containing DMEM culture medium was added to each well of 24-well plate in which collagen-adhered OMEC obtained in the above (5) was contained, and OMEC was transfected with Ad-NK4 prepared in the above (2) at 37°C for 2 hours and at 10 to 200 MOI (multiplicity of infection) per 500 μl of the culture medium. The culture supernatant was removed after transfection to obtain a cell-containing preparation (Production Example 1). Collagen gel-adhered OMEC was also transfected with E1a and E1b expressing lacZ gene of Escherichia coli, and with E3 deficient adenovirus vector (Ad-lacZ: H. Ueno, University of Occupational and Environmental Health, Fukuoka, Japan) to prepare a sample of Comparative Example 1.

(Test Example 1) Confirmation of production ability of NK4 of OMEC into which DNA encoding NK4 has been introduced

OMEC ($2 \times 10^5$ cells) was seeded in each well of a 12-well plate (Greiner Bio-one Co., Ltd.) coated with collagen type I. After adding 1 ml of 2% FBS-containing DMEM culture medium, the cells were cultivated for 72 hours, followed by transfecting with Ad-NK4 at 10, 50 and 100 MOI relative to 500 μl of the culture medium (OMEC into which Ad-NK4 is introduced). The supernatant of the culture medium was removed after the infection, and 1 ml of DMEM containing 2% FBS was added to each well. The culture supernatant was taken out at every 48 hours after the infection to measure the amount of secreted NK4 in the culture supernatant. OMEC not infected with Ad-NK4 was also cultured as a control, and the amount of secreted NK4 was measured. The concentration of NK4 was measured using IMMUNUS human HGF enzyme immunoassay kit (Institute of Immunology, Tokyo, Japan).

The results are shown in Fig. 4. The amount of secretion of NK4 was the maximum at MOI 100 and 200 in Ad-NK4-introduced OMEC, and the amount was gradually decreased independent of MOI. No secretion of NK4 was observed in OMEC into which Ad-NK4 was not introduced. Each measured value was expressed by "average value ± SD".

(Test Example 2) Confirmation of proliferation ability of OMEC into which DNA encoding NK4 was introduced

OMEC ($5\times10^4$ cells) were seeded on each well of 24-well plate coated with collagen type I. After culturing for 72 hours, OMEC was transfected with Ad-NK4 at 100 MOI (OMEC in which Ad-NK4 was introduced). The culture supernatant was removed after transfection, and 500 μl of the FEM culture medium was added to each well. After 24 or 48 hours, the cells were separated using 0.05% EDTA, and the number of the cells was counted with a cell counter (Coulter counter: Beckman Coulter Inc., CA) . OMEC not transfected with Ad-NK4 (OMEC into which no Ad-NK4 was introduced) was also cultivated as a control, and the cell number was counted. Each measured value was expressed by "mean value ± SD".

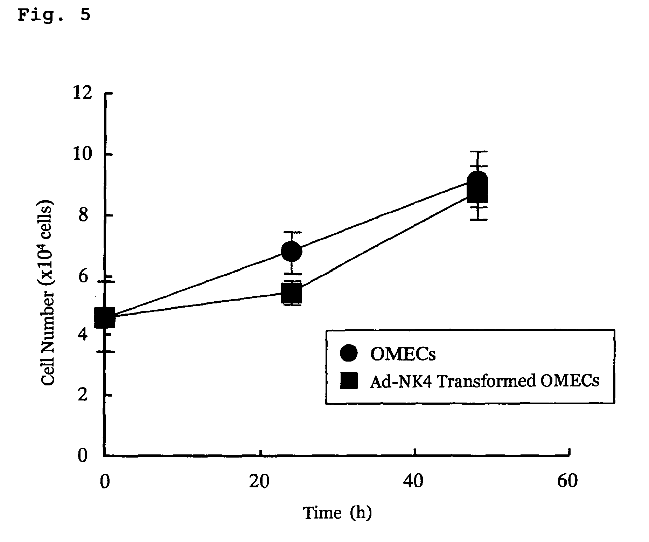

The results are shown in Fig. 5. No difference was observed between the proliferation ability of OMEC in which Ad-NK4 was introduced and that of OMEC in which Ad-NK4 was not introduced on the collagen gel. From the combined results of this Test Example 1 and Test Example 2, it was confirmed that proliferation ability on the collagen gel was not affected even through transformation of OMEC with the virus vector and secretion of NK4.

(Test Example 3) Confirmation of inhibitory effect of cancer invasion by NK4 produced from OMEC into which DNA encoding NK4 is introduced

OMEC ($1\times10^5$ cells) were seeded on 12-well plate coated with collagen type I. After 72 hours of cultivation, OMEC was transfected with Ad-NK4 100 MOI (OMEC into which Ad-NK4 was introduced). The culture supernatant was removed after the infection, and 1 ml of DMEM containing 2% FBS was added to each well. The culture supernatant was sampled 3 or 4 days after transfection, and this supernatant (NK4-sup) was used for the invasion inhibition test. Used for the invasion inhibition test was 24-well Matrigel Invasion double chamber (Becton Dikinson, Bedfold, MA).

HGF-producing human fibroblast cells (MRC-5: RIKEN Cell Bank, Ibaraki, Japan) were seeded in 10% FBS-containing DMEM contained in the outer cup of Matrigel invasion double chamber at a density of $1.5\times10^5$ cells/cm$^2$. After 24 hours of cultivation, the culture medium was replaced with 2% FBS-containing DMEM. DMEM containing 2% FBS (control) and spleen cancer cells (SUIT-2 or AsPC-1: H. Iguchi, National Kyushu Cancer Center, Fukuoka, Japan) suspended in the above NK4-sup were seeded at a density of $5\times10^4$/cup in the inner cups of Matrigel invasion double chamber, respectively. After 24 hours of cultivation, the cells were stained with hematoxin-eosin to count the number of the invaded spleen cancer cells. Five fields of vision were randomly selected under the microscope to measure the number of the invaded cells, and the number was expressed in terms of "average value ± SD".

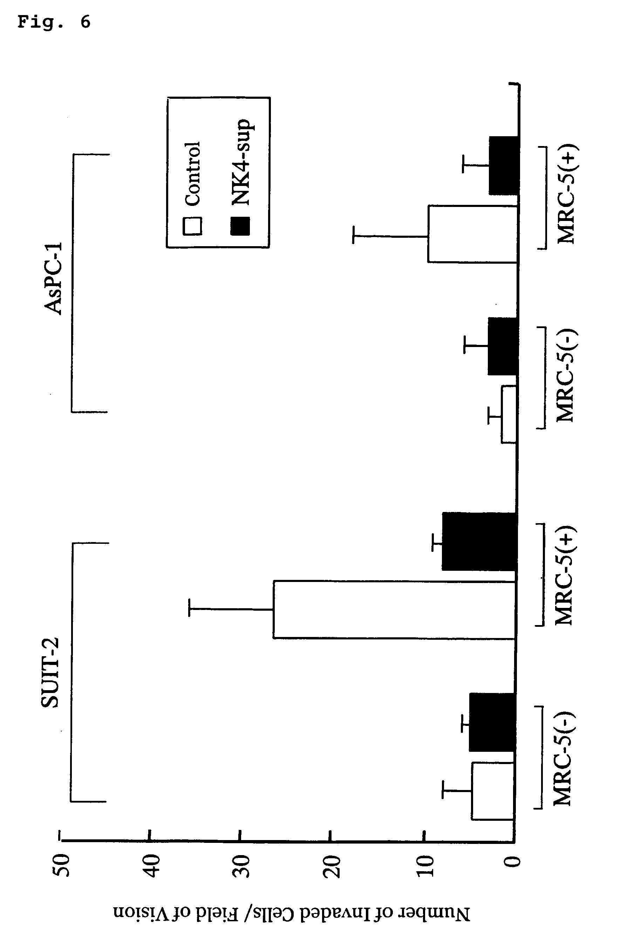

The results are shown in Fig. 6. While almost no invasion was observed in the absence of MRC-5 (MRC-5 (-)) in both the spleen cancer cells suspended in 2% FBS-containing DMEM (control) and the spleen cancer cells suspended in NK4-sup irrespective of the kind of the spleen cells, invasion of the spleen cells was observed in the presence of MRC-5 (MRC-5(+)) as compared with the result obtained in the absence of MRC-5 (MRC-5(-)), and the number of invaded cells was remarkably increased in SUIT-2 cells. In contrast, the number of the invaded spleen cancer cells was remarkably inhibited for the SUIT-2 and AsPC-1 cells when the spleen cancer cells were suspended in the NK4-sup.

(Example 1) Production of NK4 for cell-containing preparation

AsPC-1 cells ($3\times10^6$ cells) suspended in 50 μl of DMEM were transplanted into the muscle at the left flank of a 4-weeks old nude mouse (BALB/c nu/nu, Kyudo Co. , Ltd. , Saga, Japan) to form a tumor. Fourteen days after implantation of AsPC-1 cells, the cell-containing preparations prepared in Production Example 1 (Production Example 1 group: n = 3) and Comparative Example 1 (Comparative Example 1 group: n = 3) were subcutaneously transplanted respectively at the tumor-forming sites as shown in Fig. 1. A group was also prepared by transplanting only AsPC-1 cells as a control (control group: n = 3). Tumors of each nude mouse in Production Example 1 group and Comparative Example 1 group were sampled together with surrounding tissues on day 4 after implantation. The tumor sampled from each group was washed once with PBS and homogenized using RIPA buffer [1% RIPA (UPSTATE, NY), 1 mM sodium orthovanadate (Wako Pure Chemical Industries), 1 mM phenylmethylsulfonyl fluoride (Wako Pure Chemical Inductries)] to obtain an extraction solution of each tumor. The amount of NK4 in this tumor extraction solution was measured by ELISA method. The serum was also sampled from each mouse in Production Example 1 group, and the amount of NK4 in the serum was measured by the same method. Each measured value was expressed in terms of "average value ± SD".

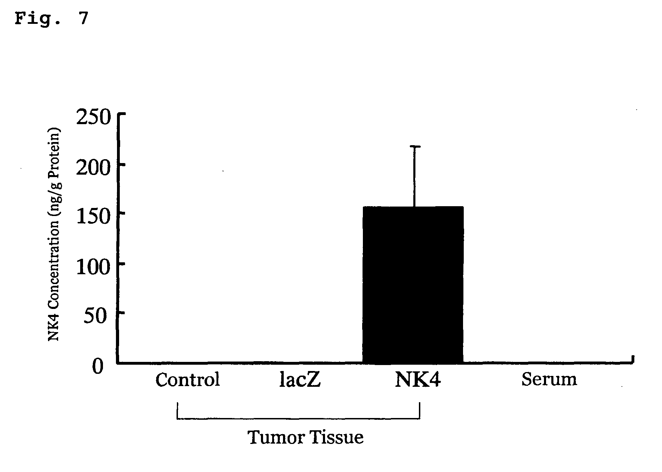

The results are shown in Fig. 7. While the amount of NK4 from the control group and Comparative Example 1 group was not more than 0.3 ng/total protein (g), the amount of NK4 in the tumor extraction solution from the Production Example 1 group was 155.85 ± 60.83 ng/total protein (g). NK4 was not detected in the serum of Production Example 1 group. It was confirmed from these results that NK4 secreted from the cell-containing preparation of Production Example 1 was supplied to the tumor of the mouse implanted with the preparation in Production Example 1, and that the NK4 was locally and efficiently supplied to the target tumor.

(Example 2) Inhibition of growth of cancer using cell-containing preparation

Tumors were formed in nude mice by the same method as in Example 1. Three days after implantation of AsPC-1 cells, the cell-containing preparation prepared in Production Example 1 (Production Example 1 group: n = 5) or Comparative Example 1 (Comparative Example 1 group: n = 5) was subcutaneously transplanted at the tumor-forming site as shown in Fig. 1. A group (control group: n = 5) in which only AsPC-1 cells were transplanted was also prepared as a control. On day 3 and 5 after implantation of the preparation in Production Example 1 or Comparative Example 1, the volume of the tumor in nude mouse was measured using a gauge (caliper). The volume of the tumor was defined by the following equation. Each measured value was expressed in terms of average value $\pm$ SD ($p < 0.05$).

$$\texttt{Volume of tumor (mm}^2\texttt{) = 0.52} \times \texttt{(width (mm))}^2 \times \texttt{length (mm)}$$

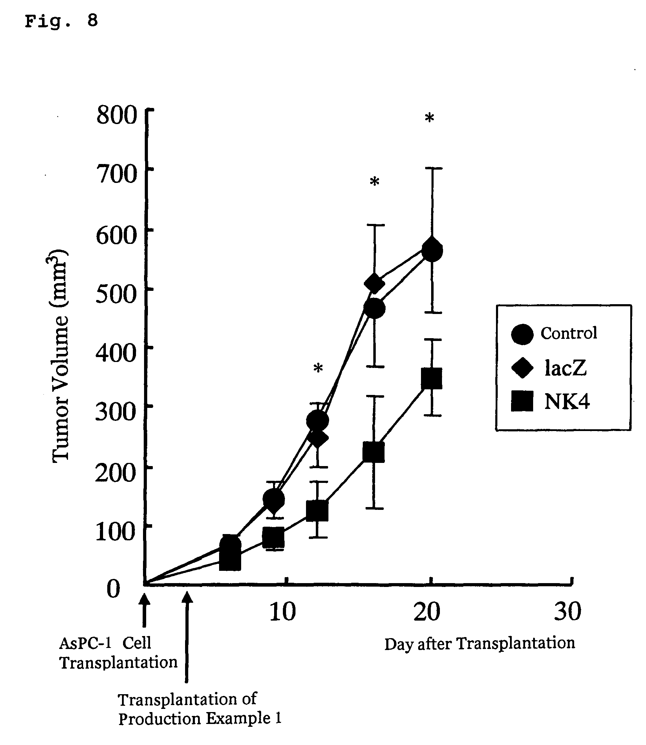

The results are shown in Fig. 8. The tumor volume was remarkably increased with the lapse of time in Comparative Example 1 group and control group. On the contrary, the increase of the tumor volume was inhibited in Production Example 1 group. These results clearly show that the cell-containing preparation according to Production Example 1 has an inhibitory effect of tumor proliferation.

(Example 3) Inhibition of angiogenesis using cell-containing preparation

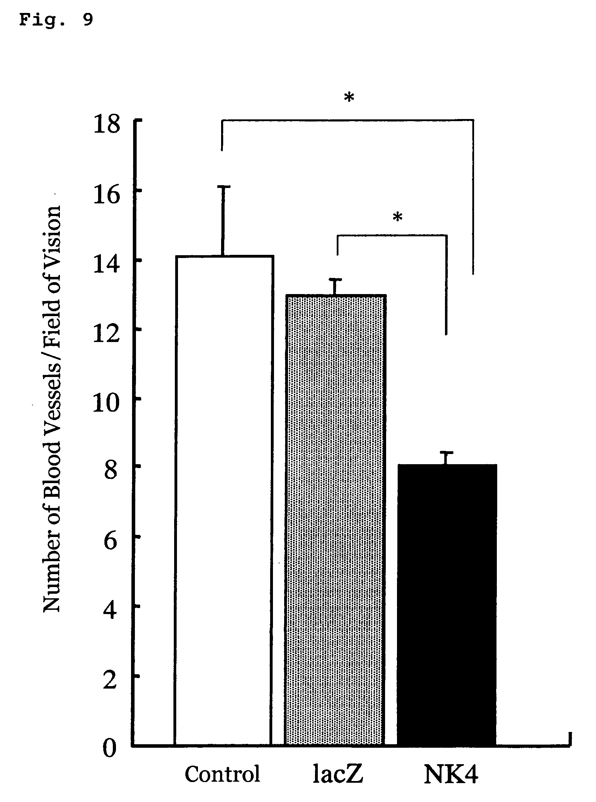

Tumors were formed in nude mice by the same method as in Example 1. Three days after implantation of AsPC-1 cells, the cell-containing preparation prepared in Production Example 1 (Production Example 1 group: n = 4) and in Comparative Example 1 (Comparative Example 1 group: n = 4) were subcutaneously transplanted at the tumor-forming site as shown in Fig. 1. A group (control group: n = 3) to which only AsPC-1 cells were implanted was also prepared as a control. On day 14 after implantation of AsPC-1 cells, the tumor sampled from each group was subjected to an immnohistochemical staining, and the number of newborn blood vessels was measured. The immnohistochemical staining will be described below. A tissue section sampled from each group was fixed with paraffin, and a rabbit polyclonal antibody against von Willebrand factor (DAKO Co., CA) was adhered to each tissue section, which was stained with DAB-peroxidase complex. The number of newborn blood vessels was counted under a microscope ($\times$ 200). Each measured value was expressed in terms of average value $\pm$ SD ($p < 0.05$).
The results are shown in Fig. 9. Remarkable angiogenesis was observed in Comparative Example 1 group and control group. On the contrary, increase of the number of newborn blood vessels was inhibited in Production Example 1 group. These results show that the preparation according to Production Example 1 has a angiogenesis inhibitory effect.

Industrial Applicability

The cell-containing preparation of the present invention is an effective therapeutic agent for inhibiting the metastasis of primary tumors or cancers, and is safe to the body. In particular, NK4 having actions for inhibiting the growth or metastasis of cancers can be more efficiently supplied to cancer cells according to the present invention. The present invention provides a method for inhibiting growth of primary tumors, a method for inhibiting metastasis of cancers, or a method for inhibiting angiogenesis using the cell-containing preparation of the present invention.

**Claims**

1. A cell-containing preparation comprising a cell which has a DNA having a base sequence represented by SEQ ID NO: 1 or 2 or a DNA hybridizable with a DNA having a base sequence represented by SEQ ID NO: 1 or 2 under stringent conditions and a fibrous protein.

2. The cell-containing preparation according to claim 1, wherein the cell is an epithelial cell of the oral mucosa, a skin cell or a fibroblast.

3. The cell-containing preparation according to claim 1 or 2, wherein the fibrous protein is collagen.

4. The cell-containing preparation according to any one of claims 1 to 3, wherein the cells are deposited on the surface of the fibrous protein.

5. The cell-containing preparation according to any one of claims 1 to 4, wherein the cell is a transformant.

6. The cell-containing preparation according to claim 5, wherein the transformant is transformed with a recombinant expression vector.

7. The cell-containing preparation according to claim 6, wherein the recombinant expression vector is adeno-associated virus (AAV), retrovirus, poxvirus, herpes virus, herpes simplex virus, lentivirus (HIV), Sendai virus, Epstein-Barr virus (EBV), vaccinia virus, polio virus, sindbis virus, SV40 or plasmid.

8. The cell-containing preparation according to any one of claims 1 to 7 capable of forming a peptide encoded by a DNA having a base sequence represented by SEQ ID NO: 1 or 2, or by a DNA hybridizable with a DNA having a base sequence represented by SEQ ID NO:1 or 2 under stringent conditions.

9. The cell-containing preparation according to any one of claims 1 to 8 further containing a mesh sheet comprising a biodegradable resin.

10. The cell-containing preparation according to claim 9, wherein the biodegradable resin is polyglycolic acid.

11. The cell-containing preparation according to any one of claims 1 to 10, which is an anticancer agent or a cancer metastasis inhibitor.

12. The cell-containing preparation according to claim 11, which is an anticancer agent or a metastasis inhibitor for ovarian cancer, pancreatic cancer, stomach cancer, gall bladder cancer, kidney cancer, prostate cancer, breast cancer, esophageal cancer, liver cancer, oral cavity cancer, colon cancer, large intestine cancer, sarcoma, glioma or melanoma.

13. The cell-containing preparation according to any one of claims 1 to 10, which is an angiogenesis inhibitor.

14. A method for inhibiting growth, invasion and metastasis of cancers or for inhibiting angiogenesis, which comprises administering the cell-containing preparation according to any one of claims 1 to 13 to a mammal.

15. A method for producing a cell-containing preparation, which comprises culturing a cell on the surface of a fibrous protein and transforming the cultured cells with a recombinant expression vector comprising a DNA having a base sequence represented by SEQ ID NO:1 or 2, or with a recombinant expression vector comprising a DNA hybridizable with a DNA having a base sequence represented by SEQ ID NO:1 or 2 under stringent conditions.

16. A method for producing a cell-containing preparation, which comprises preparing a fibrous protein sheet by coating a fibrous protein onto a mesh sheet comprising a biodegradable resin; culturing a cell on the surface of the fibrous protein sheet obtained; and transforming the cultured cells with a recombinant expression vector comprising a DNA having a base sequence represented by SEQ ID NO:1 or 2, or with a recombinant expression vector comprising a DNA hybridizable with a DNA having a base sequence represented by SEQ ID NO: 1 or 2 under stringent conditions.

17. A method for producing a cell-containing preparation, which comprises transforming the cells with a recombinant expression vector comprising a DNA having a base sequence represented by SEQ ID NO: 1 or 2, or with a recombinant expression vector comprising a DNA hybridizable with a DNA having a base sequence represented by SEQ ID NO: 1 or 2 under stringent conditions, and mixing the resulting transformant cells with a fibrous protein.

Fig. 1

Compacting

Fig. 2

Envelope Signal and Enhancer

Cytomegalovirus Initial Enhancer/Promoter

NK4 cDNA

SV40 Polyadenylation Signal

Ad-NK4

pJM17
(Vector Based on
Adenovirus Type 5 Genomes)

Fig. 3

← 3

← 1

← 2

Fig. 4

Day after Transformation

Fig. 5

Fig. 6

Fig. 7

Fig. 8

Fig. 9

# EP 1 642 586 A1

## INTERNATIONAL SEARCH REPORT

International application No.

PCT/JP2004/000630

### A. CLASSIFICATION OF SUBJECT MATTER
Int.Cl$^7$ A61K35/12, A61K38/39, C12N5/10, A61P35/00

According to International Patent Classification (IPC) or to both national classification and IPC

### B. FIELDS SEARCHED

Minimum documentation searched (classification system followed by classification symbols)
Int.Cl$^7$ A61K35/12, A61K38/39, C12N5/10, A61P35/00

Documentation searched other than minimum documentation to the extent that such documents are included in the fields searched

Electronic data base consulted during the international search (name of data base and, where practicable, search terms used)
WPIDS/BIOSIS/BIOTECHABS/MEDLINE/CA(STN)

### C. DOCUMENTS CONSIDERED TO BE RELEVANT

| Category* | Citation of document, with indication, where appropriate, of the relevant passages | Relevant to claim No. |
|---|---|---|
| Y | Keiji KUBA "HGF ni yoru Kikan Saisei to NK4 ni yoru Seigan", Analytikem International, Inc., 2002, Vol.20, pages 2143 to 2450 | 1-13,15-17 |
| Y | Tadanori MAYUMI "Saisei Eryo to DDS Overview – Yakugakuteki Shiten Kara-", Drug Delivery System, 2001, Vol.16, pages 10 to 15 | 1-13,15-17 |
| Y | Tadanori MAYUMI "Yakubutsu Gainen no Paradigm Shift -Ko Bunshi Eyakuhin ni yoru Sippei Chiryo ni Mukete-", Journal of Japanese Cosmetic Science Society Gakkai Shi, 2002, Vol.26, pages 234 to 238 | 1-13,15-17 |

☒ Further documents are listed in the continuation of Box C. ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 05 April, 2004 (05.04.04) | 20 April, 2004 (20.04.04) |

| Name and mailing address of the ISA/ | Authorized officer |
|---|---|
| Japanese Patent Office | |
| Facsimile No. | Telephone No. |

Form PCT/ISA/210 (second sheet) (January 2004)

23

**EP 1 642 586 A1**

**INTERNATIONAL SEARCH REPORT**

International application No.

PCT/JP2004/000630

C (Continuation). DOCUMENTS CONSIDERED TO BE RELEVANT

| Category* | Citation of document, with indication, where appropriate, of the relevant passages | Relevant to claim No. |
|---|---|---|
| Y | WO 92/09702 A (New York University), 30 April, 1992 (30.04.92), & EP 554352 A1 & JP 6-502406 A & US 20020009431 A1 | 1-13,15-17 |
| Y | WO 00/61156 A2 (Titan Pharmaceuticals, INC.), 19 October, 2000 (19.10.00), & EP 1165100 A2 & JP 2003-532621 A | 1-13,15-17 |
| Y | Yoshito IKADA, Gairon Protein, Nucleic acid and Enzyme, 2000, Vol.45, pages 2139 to 2141 | 1-13,15-17 |
| Y | Tetsuji YAMAOKA et al., "Seitainai Kyushusei Zairyo no Bunshi Sekkei", Protein, Nucleic acid and Enzyme, 2000, Vol.45, pages 2142 to 2149 | 1-13,15-17 |
| P,X | MANABE T. et al., Cell-based protein delivery system for the growth of pancreatic cancer: NK4 gene-transduced oral mucosal epithelial cell sheet, Cliniccal Cancer Research, August, 2003, Vol.9, pages 3158 to 3166 | 1-13,15-17 |

Form PCT/ISA/210 (continuation of second sheet) (January 2004)

24

**EP 1 642 586 A1**

<div align="center">

**INTERNATIONAL SEARCH REPORT**

</div>

| International application No. |
| --- |
| PCT/JP2004/000630 |

| Box No. I    Nucleotide and/or amino acid sequence(s) (Continuation of item1.b of the first sheet) |
| --- |

1. With regard to any nucleotide and/or amino acid sequence disclosed in the international application and necessary to the claimed invention, the international search was carried out on the basis of:

    a.   type of material

        [X]  a sequence listing

        [ ]  ·table(s) related to the sequence listing

    b.   format of material

        [ ]  in written format

        [X]  in computer readable form

    c.·  time of filing/furnishing

        [ ]  contained in the international application as filed

        [X]  filed together with the international application in computer readable form

        [ ]  furnished subsequently to this Authority for the purposes of search

2. [X]  In addition, in the case that more than one version or copy of a sequence listing and/or table relating thereto has been filed or furnished, the required statements that the information in the subsequent or additional copies is identical to that in the application as filed or does not go beyond the application as filed, as appropriate, were furnished.

3. Additional comments:

Form PCT/ISA/210 (continuation of first sheet (1)) (January 2004)

## INTERNATIONAL SEARCH REPORT

International application No.

PCT/JP2004/000630

**Box No. II**      Observations where certain claims were found unsearchable (Continuation of item 2 of first sheet)

This international search report has not been established in respect of certain claims under Article 17(2)(a) for the following reasons:

1. ☒ Claims Nos.: 14
   because they relate to subject matter not required to be searched by this Authority, namely:
   It pertains to methods for treatment of the mammal body including human body by surgery or therapy and diagnostic methods.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III**      Observations where unity of invention is lacking (Continuation of item 3 of first sheet)

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**      ☐ The additional search fees were accompanied by the applicant's protest.

☐ No protest accompanied the payment of additional search fees.

Form PCT/ISA/210 (continuation of first sheet (2)) (January 2004)